# EUROPEAN PATENT SPECIFICATION

(11) **EP 0 467 966 B1**
(45) Date of publication and mention of the grant of the patent: **19.06.1996**
(21) Application number: 90906629.2
(22) Date of filing: 11.04.1990
(51) Int. Cl.: A61N 1/04, A61B 5/0408

(54) **ELECTRICAL STIMULATION ELECTRODE WITH IMPEDANCE COMPENSATION**
ELEKTRISCHE REIZELEKTRODE MIT IMPEDANZAUSGLEICH
ELECTRODE DE STIMULATION ELECTRIQUE A COMPENSATION D'IMPEDANCE

(30) Priority: 13.04.1989 US 337389
(43) Date of publication of application: 29.01.1992
(73) Proprietor: Axelgaard, Jens, Fallbrook, California 92028 (US)
(72) Inventor: AXELGAARD, Jens, Fallbrook, CA 92028 (US); GRUSSING, Theodore, Huntington Beach, CA 92646 (US)
(74) Representative: KOHLER SCHMID + PARTNER
(86) International application number: US9001975
(87) International publication number: WO9011796

(56) References cited:
- EP-A- 0 226 568
- US-A- 4 243 051
- US-A- 4 243 052
- US-A- 4 722 354
- US-A- 4 867 166
- US-A- 4 893 626

## Description

The present invention generally relates to electrodes and, more particularly, to electrodes suitable for transcutaneous nerve and/or muscle stimulation of the kind described in the preambles of independent claims 1 and 13.

Continued development of electrical medical devices has produced a need for a variety of electrodes.

Although many of these electrodes have, as a design objective, good electrical signal transmission between a patient's skin surface and electrical leads interconnected with a device, each has specific requirements dependent upon the type of apparatus for which it is to be used.

As an example, electrocardiograph (EKG) and electroencephalograph (EEG) machines are primarily monitoring type devices which require small contact surfaces, or area, with the patient's skin.

On the other hand, transcutaneous electric nerve stimulation (TENS), and muscle stimulation devices require relatively large skin surface contact to effect such nerve and muscle stimulation.

Transcutaneous electrical nerve stimulation is useful, for example, in post-operative and chronic pain control, while muscle stimulation is useful, for example, in maintaining and development of muscle tissue. Electrodes suitable for use in nerve and muscle stimulation preferably provide a uniform electrical coupling across the skin electrode interface.

As hereinbefore noted, electrodes suitable for nerve and/or muscle stimulation may be relatively large having dimensions of several inches or more.

Because nerve and/or muscle stimulation causes muscle contraction, a considerable amount of skin movement is associated therewith.

Additionally, perspiration from the skin is more likely to loosen or disrupt the electrode because of its large size. As should be apparent, the larger the electrode, the longer the evaporation path, or distance, the perspiration occurring at the center regions of the electrode must travel in order to evaporate, or be released to the atmosphere.

It has been found that prior art electrodes which have been secured to the surface of a patient's skin with medical adhesive tape, or the like, have a tendency to lift off from the skin because of perspiration and movement of the patient's skin during treatment.

Because an electrode suitable for nerve and/or muscle stimulation must provide for an electrical signal to be distributed over the entire surface of the electrode, the electrode must necessarily be conductive.

Prior art electrodes have utilized a number of conductive elements, such as carbon impregnated rubber and vinyl, as well as metallic foils.

However, a useful electrode must be flexible in order to accommodate relative movement of the patient's skin therebeneath, as hereinabove-described.

Because nerve and muscle stimulation electrodes may be utilized over a long period of time, as may be necessary in connection with sports injuries, the electrode must be compatible with the skin and flex therewith.

Insufficient flexing of the electrode can result in severe irritation of the patient's skin and electrical "hot spots" due to uneven electrode-skin contact, which manifests itself in a rash and a burning sensation.

The sensation of burning may be sensed by the patient within a few minutes after application of electrical signals during nerve and/or muscle stimulation, while the rash conditions generally take a longer period of time to develop.

It has been found that the use of prior art electrodes in nerve and/or muscle stimulation results in a skin rash in up to 25% to 35% of the people undergoing treatment.

An additional problem associated with the necessary stretchability of electrodes utilized in nerve and/or muscle stimulation procedures is that while the electrode must be able to flex, or stretch, in order to accommodate skin movement during treatment, the conductivity of the electrode should not be interrupted, or distorted, due to the stretching of the electrode.

Prior art electrodes have compromised the flexibility of the electrode in an effort to provide uniform current densities over the entire contact area of the electrode. These electrodes typically utilize a metallic mesh, or foil, to provide contactivity of the electrode and utilize a conductive gel between the electrode and the patient's skin in order to accomodate movement therebetween.

There is, however, relative movement between the relatively rigid electrode and the skin, which is accommodated for by the gel. This relative movement oftentimes causes the gel to move from beneath the conductive portion of the electrode, thereby limiting the useful life of the electrode on the skin.

In addition, this relative motion between the skin and the electrode does not provide for the maintenance of the position of the electrode relative to the nerve and/or muscle being stimulated.

Precision positioning of the electrode is, of course, performed by a physician, or the like, knowledgeable in the treatment method. Inaccurate placement of the electrode, or slipping of the electrode from its intended position, may significantly reduce the beneficial effects of the treatment.

Hence, there is a need for a flexible electrode for use with electrical stimulation devices which adheres well to the patient's skin, is easily removed therefrom, and is able to move with the patient's skin in order to ensure proper continuous placement of the electrode relative to nerve or muscle tissue being stimulated, as well as providing long-term continuous electrical connection therewith without irritation of the skin or discomfort to the patient under treatment. The electrode of the present invention fulfills these needs.

US patent manuscript 4,722,354 describes a flexible transcutaneous electrical nerve and muscle stimulation electrode having a stretchable conductive material for coupling electrical signals to a patient's skin, and a flexible solid conductive adhesive disposed on one side of the conductive material for adhering the flexible transcutaneous electrical nerve and muscle stimulation electrode to the skin of a patient and for providing an electrical conducting contact therebetween. A non-conductive sheet is disposed on another side of the conductive material for preventing undesired electrical contact with the conductive material. This configuration has the disadvantage that the stretchable conductive material exhibits a higher resistivity along one direction of the material than along a transverse direction thereof, so that an uneven electrical impulse distribution and consequently an uneven treatment of the underlying nerves and muscles across the surface area of the electrode occurs.

In view of the above mentioned prior art, the technical problem addressed by the invention is improving the electrical properties of the electrode for reducing irritation to the skin and patient discomfort.

### SUMMARY OF THE INVENTION

This problem is solved by the characterizing portions of claims 1 and 14 in combination with their respective preambles.

A flexible transcutaneous electrical nerve and/or muscle stimulation electrode in accordance with the present invention includes a conductive material for directly coupling electrical signals to a patient's skin. In addition, the conductive material may comprise a conductive fabric having conductive fiber means for enabling the conductive fabric to be stretched. The conductive fabric comprises an array of conductive fibers with interstitial areas therebetween and has a different electrical resistivity in different directions therealong. Electrical shunt means are provided and disposed along the conductive fabric for causing more uniform resistance between any two equally spaced points on said conductive fabric than without the electrical shunt.

Flexible conductive adhesive means are provided and disposed within the interstitial areas and on one side of the conductive fabric for adhering the flexible transcutaneous electrical nerve and/or muscle stimulation electrode to the skin of a patient and providing an electrical conducting contact therebetween.

Interconnection with an electrical stimulation device is provided by means of an electrical lead wire which is interconnected with the electrical shunt means and a non-conductive sheet is disposed on the conductive fabric for preventing undesired electrical contact therewith.

More particularly, the flexible stimulation electrode according to the present invention may include a conductive fabric comprising a knit of conductive fibers having a higher resistivity along a longitudinal direction of knit than along a transverse direction of knit and the resistivity along the longitudinal direction may be up to 20 times the resistivity along the transverse direction of knit. The shunt, in accordance with the present invention, may include a single wire or a plurality of strands.

In one embodiment of the present invention, the electrical shunt includes a plurality of strands disposed in a vein-like manner having a dichotomous venation.

Another embodiment of the present invention, the electrical shunt may include a plurality of strands disposed in a vein-like manner having a pinnate reticulate venation.

And yet, another embodiment of the present invention the electrical shunt may include a plurality of strands disposed in a vein-like manner having a palmate reticulate venation.

Still another embodiment of the present invention may include electrical shunt including a plurality of strands disposed in a vein-like manner having a parallel venation. The electrical shunt means may be disposed on an opposite side of the conductive fabric and held against the opposite side in an electrically conductive manner by the flexible solid conductive adhesive disposed in the interstitial areas.

Means may also be provided defining an overall shape of the conductive fabric for enabling improved conformity around and/or between body extremities than a conductive fabric having a shape selected from the group of square, rectangular and round. This overall shape results in a different electrical resistance between any two opposing points along a perimeter of the conductive fabric. In this embodiment, electrical shunt means are provided and disposed along the conductive fabric for equalizing the electrical resistance between any two points along the perimeter of the conductive fabric.

### DESCRIPTION OF THE DRAWINGS

The advantages and features of the present invention will be better understood by the following description and drawings in which:
Figure 1 is a perspective view of a flexible transcutaneous electrical nerve and/or muscle stimulation electrode in accordance with the present invention showing its disposition on a patient's skin;
Figure 2 is a perspective view of the stimulation electrode with a portion of a non-conductive sheet thereof peeled back to show an electrical lead wire therein;
Figure 3 is a cross-sectional view of the transcutaneous electrical nerve and/or muscle stimulation electrode generally showing conductive fabric, a flexible conductive adhesive, electrical lead wire, non-conductive sheet and a pressure sensitive adhesive;
Figure 4 is an enlarged view of the conductive fabric utilized in the present invention generally showing a honeycomb latch needle knit;
Figure 5 is an illustration of the conductive fabric utilized in the present invention stretched in a transverse direction;
Figure 6 is an illustration of the conductive fabric utilized in the present invention stretched in a longitudinal direction;
Figure 7 is an illustration of woven conductive fabric suitable for use in the present invention;
Figure 8 is a plan view of a transcutaneous electrical nerve and/or muscle stimulation electrode in accordance with the present invention having an overall shape resembling a bow tie and showing an electrical shunt disposed thereon;
Figure 9 is a plan view of a flexible transcutaneous electrical nerve and/or muscle stimulation electrode having an oval shape;
Figure 10 is a plan view of a flexible transcutaneous electrical nerve and/or muscle stimulation electrode having an electrical shunt with strands disposed in a vein-like manner with dichotomous venation;
Figure 11 is a plan view of a flexible transcutaneous electrical nerve and/or muscle stimulation electrode with pinnate reticulate venation;
Figure 12 is a plan view of a flexible transcutaneous electrical nerve and/or muscle stimulation electrode with palmate reticulate venation; and
Figure 13 is a plan view of a flexible transcutaneous electrical nerve and/or muscle stimulation electrode with parallel venation.
Figure 14a and b are plan views of a 2 inch x 5 inch electrode each having different shunt lengths with impedance measurement portions shown thereon;
Figure 15 is a probe map of the electrode shown in Figure 14a;
Figure 16 is a probe map of the electrode shown in Figure 14b.
Figure 17 is a probe map of an oval electrode with a 1.2 inch shunt; and
Figure 18 is a probe map of an oval electrode with a 4 inch shunt.

### DETAILED DESCRIPTION

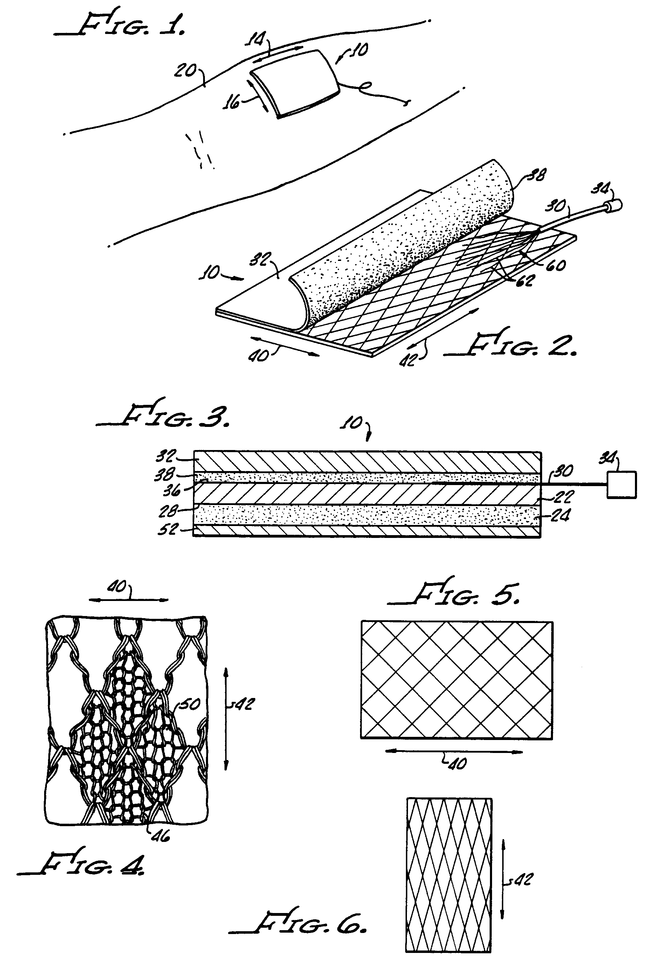

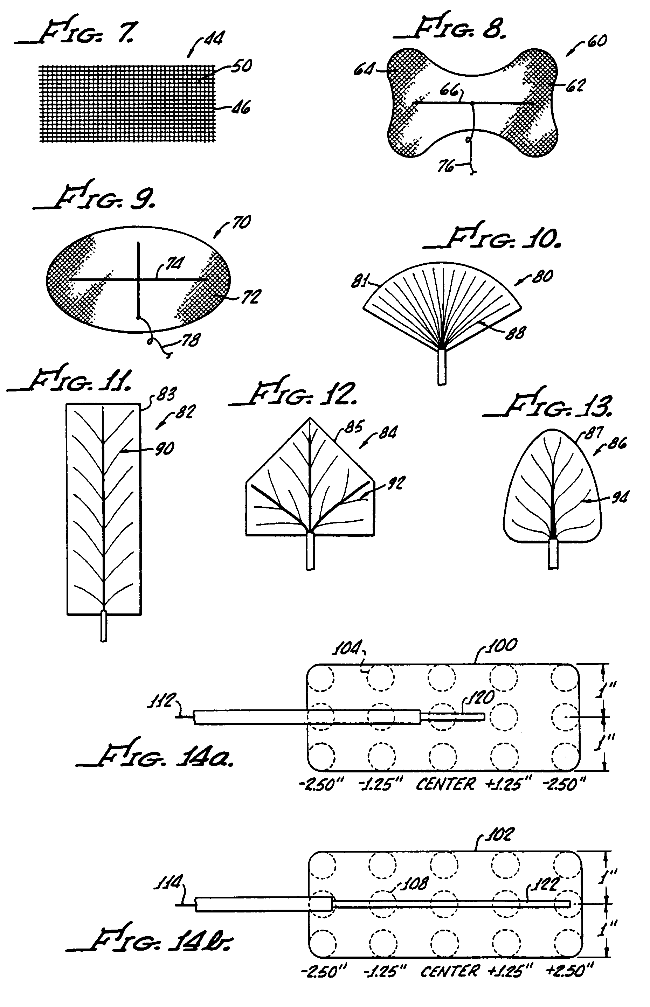

Turning now to Figures 1 and 2, there is shown, in perspective view, a flexible transcutaneous electrical nerve and/or muscle stimulation electrode 10 in accordance with the present invention.

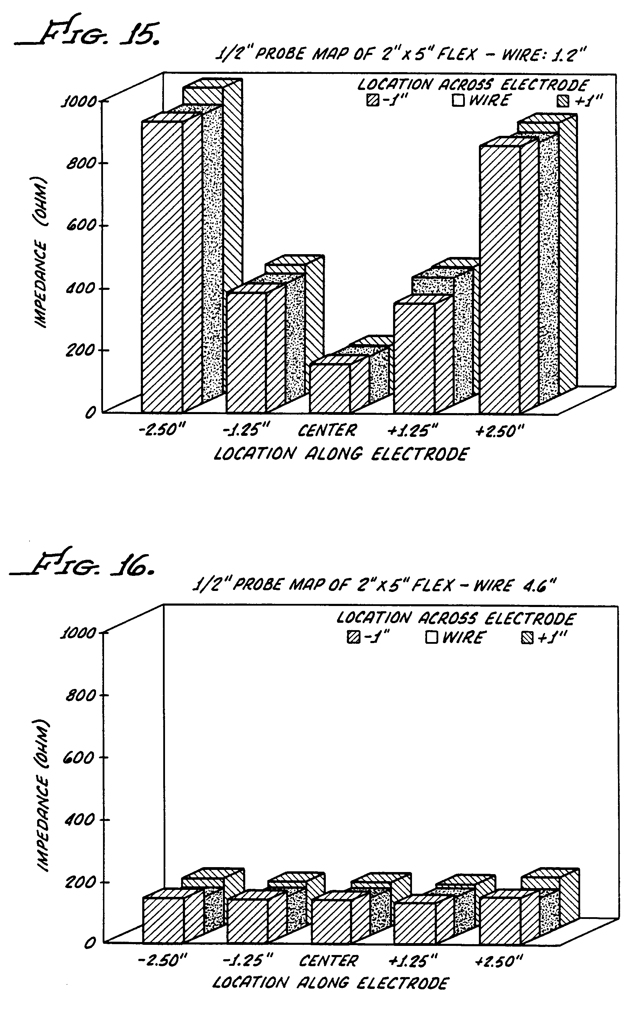

As shown in Figure 1 and hereinafter described in greater detail, the electrode 10 is flexible in two directions, as indicated by arrows 14, 16, while in place on a patient's limb 20, or body, not shown. As more clearly shown in Figure 3, the electrode 10 includes a stretchable conductive fabric 22, flexible conductive adhesive 24, which is disposed on one side 28 of the conductive fabric 22 for adhering the flexible transcutaneous electrical nerve and/or muscle stimulation electrode 10 to the skin of a patient (not shown in Figure 3) and an electrical lead wire 30 interconnected with the conductive fabric 22, as hereinafter described, for providing electrical signals to the conductive fabric 22 when interconnected with an electrical stimulation device, not shown, by means of a connector 34, or the like.

In addition, a non-conductive sheet, such as a flexible plastic 32 disposed on another side 36 of the conductive fabric 22 by means of a pressure sensitive adhesive 38, provides means for preventing undesired electrical contact with the conductive fabric 22, as may occur during wearing of the device.

It should be appreciated that the conductive fabric 22 must be isolated from outside objects and other areas of the patient's skin in order to preferentially couple electrical signals into the patient's body where prescribed by an attending physician.

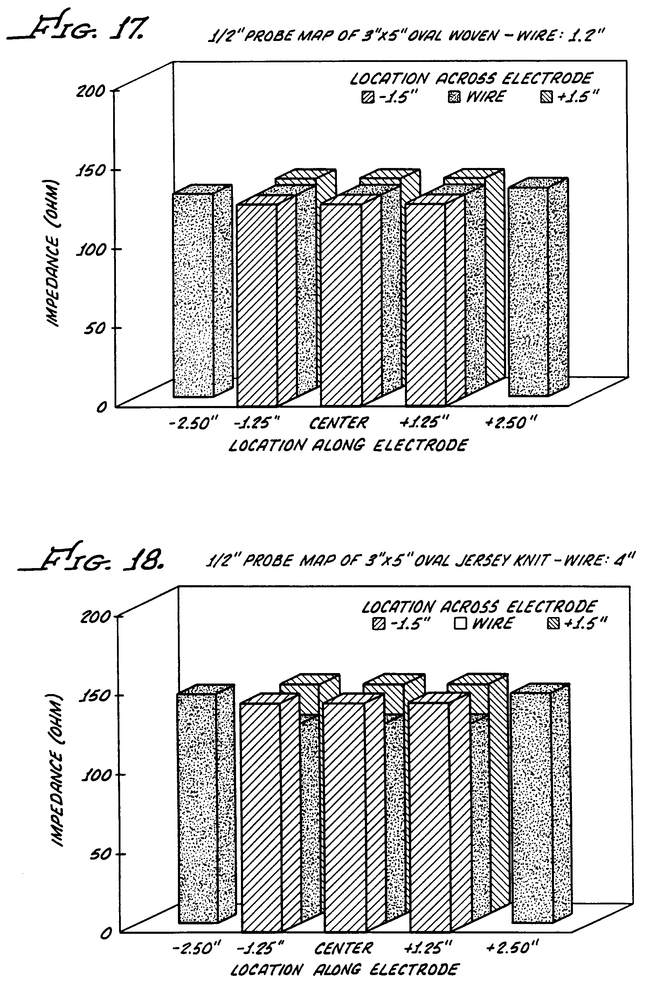

It has been found that a knit fabric, preferably a one-quarter-inch honeycomb latch needle knit fabric, as depicted in Figure 4, provides for a fabric which may be stretched up to about 100 percent greater than a first original conductive fiber dimension in the direction of stretch, see arrow 40 and Figure 5, and up to about 50 percent greater than a second original fabric dimension in a second direction of stretch, see arrow 42 and Figure 6, without loss of conductivity of the fabric. Knits of this nature are commercially available from knitters, such as, for example, Paragon West Knitting Mill in Anaheim Hills, California

Woven fabrics, such as illustrated in Figure 7, are also suitable if less stretch is required, as may be the case when the electrode is utilized on less curvature portions of the body, such as the back.

The conductivity of the fabric is provided by the individual conductive fibers 46. It has been found that a conductive fiber No. BK50/2 manufactured by Bekaert of Belgium, which includes a blend of 20 percent 316 stainless steel and 80 percent of polyester when latch needle honeycomb knitted to a density of about 2.5 pounds per square yard, produces a conductive double-stretch knit which is particularly suitable for transcutaneous nerve and/or muscle stimulation electrodes.

The double-stretch nature of the knit fabric, when incorporated into the electrode of the present invention, as hereindescribed, provides for an electrode which is contourable to the shape of a patient's body or limb.

This is particularly important with relatively large stimulation/electrodes in accordance with the present invention. The electrode 10 may have dimensions in the range of, for example, 2 inches by 3 inches, hence, the electrode must be "fitted" by stretching of the electrode 10 to the skin 20 of a patient in order to provide a uniform contact therebetween.

It is particularly important that the electrode 10 and, of course, the conductive fabric 22, do not degrade during constant and repetitious movement and stretching thereof, as the electrical signals activate muscles and nerves within the patient's body which result in continued movement, or contraction, of the skin. Because the conductive fabric is a loose knit, stretching thereof does not deteriorate any of the conductive fibers therein to any substantial degree, thus causing loss of conductivity of the electrode.

In order to be effective in transmitting electrical signals to the patient's skin 20, the electrode 10 utilizes a conductive adhesive 24, such as one manufactured by Valley Lab, Inc., of Boulder, Colorado, under the name Polyhesive, or Promeon RG-62D, manufactured by Promeon Corporation of Minneapolis, Minnesota. These proprietary products are useful in a number of electrode applications and have the advantage of being flexible so as to allow movement with the conductive fabric without losing contact with the patient's skin, or interrupting the electrical signals transmitted therethrough.

In the manufacture of the electrode 10, the conductive adhesive 24 is laminated as a gel or poured onto the surface 28 in a liquid form, whereupon it fills the interstitial areas 50 of the conductive fabric 22.

Thereafter, the adhesive is set into a gel-like material, which has good adhesion to the patient's skin, and is releasable therefrom without the annoyance of hair-pulling and the like. The conductive adhesive 24 is commercially available and is compatible with the skin in that it produces no irritation thereof.

Because the Polyhesive conductive adhesive 24 is in itself flexible, it does stretch with the conductive fabric between the interstitial areas 50 defined by the fibers 46.

Turning to Figures 2 and 3, the non-conductive plastic, or backing layer, 32 is adhered to the other side 36 of the conductive fabric 22, and both the backing layer and the pressure sensitive adhesive 38 hold the lead wire 30 in physical and electrical contact with the conductive fabric. In order to enhance contact therebetween, the conductive lead 30, which may be stranded stainless steel, having an end portion 54. The lead wire may have up to 1,159 strands of 8 micron diameter. Such wire is manufactured by Brunswick of DeLand, Florida.

In manufacture, the conductive lead is placed on the conductive fabric 22 for a distance of about one-third the length thereof. Thereafter, the backing layer 32, with adhesive 36 applied thereto, may be firmly placed over the frayed portion 60 and bonded by pressure applied thereto.

This relatively simple method of contacting the lead wire 30 with the conductive fabric 22 enables some movement therebetween as the conductive fiber and electrode stretch.

It should be appreciated that stretching along the direction 40, the major direction of stretch, may stretch the frayed strands 62 apart from one another, thus reducing the relative motion between the frayed end of 60 and the conductive fiber 22.

Because the conductive adhesive 24 is subject to drying, a release liner 52 may be provided for storage of the electrode 3 before and after use. This liner may be of any suitable plastic, or silicon-coated paper, which is strippable from the conductive adhesive 24 without disturbing the integrity of the conductive adhesive.

Turning now to Figure 8, there is shown a flexible transcutaneous nerve and/or muscle stimulation electrode 60 in accordance with the present invention, which includes a conductive fabric 62 having an overall shape of a bow-tie, or butterfly, which is knit or woven with conductive fibers 64, as hereinbefore described. In addition, an electrical shunt 66 may be provided for causing more uniform resistance between any two equally spaced apart points on the conductive fabric 62 than without the electrical shunt means. When a knit fabric is utilized, the conductive fabric may have a higher resistivity along a longitudinal direction 42 of knit than along a transverse direction 40 of knit, see Figure 4. This diversity in resistivity may amount to a factor of about 20.

Figure 9 shows an electrode 70 having a conductive fabric 72 with an overall oval shape and an electrical shunt 74 in the shape of a cross. The electrical shunt may comprise a plurality of strands, as for example, stainless wire having a diameter of about 8 microns. The shunt 66 and 74 are interconnected with lead wire 76, 78, respectively, for interconnection with an electrical stimulation device (not shown).

It should be appreciated that the shunt 66 and lead wire 76 may be continuous or separate stranded wires which are electrically connected. Another important feature of the T shunt shown in Figure 8 is the fact that the orthogonal relationship between the shunt 66 and lead wire 76 provides greater resistance to the inadvertent "pulling out" of the lead wire due to abusive handling of the electrode by the lead wires. As hereinabove mentioned, the electrical shunt 66, 74 may be comprised of multiple strands in order to accommodate for variation in electrical resistivity of the fabric and, in addition, variation in electrical resistance between any two opposing points along the perimeter of the conductive fabric caused by a particular shape of the electrode.

The stranded shunt may be disposed in a vein-like pattern as shown in Figures 10, 11, 12 and 13, for various electrodes 80, 82, 84, 86, each having a particular venation of strands 88, 90, 92, 94, selected in order to cause equal resistance across the electrodes 80, 82, 84, 86. In particular, the venation shown in Figures 10, 11, 12 and 13 may be dichotomous, pinnate reticulate, palmate reticulate or parallel.

In addition, the strands 88, 90, 92, 94 may be terminated at a given distance from electrode edges 81, 83, 85, 87, calculated to increase the edge impedance in order to eliminate or substantially reduce the unwanted effect of high current density at the edges. This results in a smooth roll-off of stimulus intensity (and associated pain sensation) from electrode site on the user to non-stimulated surroundings.

It is to be appreciated that each of the strands 88, 90, 92, 94, which may be themselves single or multiple strands, may be electrically connected by simple overlaying of the strands with one another. The non-conductive plastic, or sheet 38 (see Fig. 3), holds the strands in position.

Typical fabric impedance are shown in Table 1 for various fabrics formed from the yarn having a 18 percent stainless steel and 82 percent polyester content. As can be determined therefrom, a woven fabric has an almost uniform impedance gradient

**Table 1**

| FABRIC IMPEDANCE GRADIENTS | | | | |
|---|---|---|---|---|
| | Woven | Diamond Knit | Jersey Knit | Conductive Adhesive RG-62D |
| Longitudinal (Ohm*cm) X | 2.3 | 68.3 | 113.3 | 886.4 |
| Transverse (Ohm*cm) Y | 2.2 | 14.7 | 11.3 | 886.4 |
| X-Y Ratio | 1.0 | 4.6 | 10.0 | 1.0 |
| Yarn: 18% Stainless Steel 82% Polyester | | | | |

along both the longitudinal and transverse directions. Unfortunately, it is not as flexible as knitted fabric and intends to fray, limiting its use to round shapes. The diamond knit fabric illustrated in Figure 4, conducts about five times better in the transverse direction than in the longitudinal direction and a jersey knit fabric conducts 10 times better in the transverse direction than in the longitudinal direction. It should be appreciated that in a knitted fabric, the thread runs unbroken in the transverse direction. However, in the longitudinal direction, as the fabric comes off the knitting machine, electrical conduction is from thread to thread via knitted loops which give rise to the higher impedance. Thus, when conditions warrant the use of a knitted fabric for its greater flexibility and variation in shape, the inherent non-uniform impedance thereof poses a problem in efforts to provide uniform electric field across the entire electrode.

Figure 14a and 14b show 2 x 5 inch electrodes 100, 102, fabricated with jersey knit, the electrode 100 shown in Figure 14a having a 1.2 inch exposed lead wire 120 and the electrode 102 shown in Figure 14b having a 4.6 inch exposed lead wire 122. A 1/2 inch diameter probe was used to measure impedance from the conductive adhesive side between various positions 104, 108 and the wire 112, 114, respectively, and the results are shown in Figures 15 and 16 and numerically repeated in Tables 2 and 3.

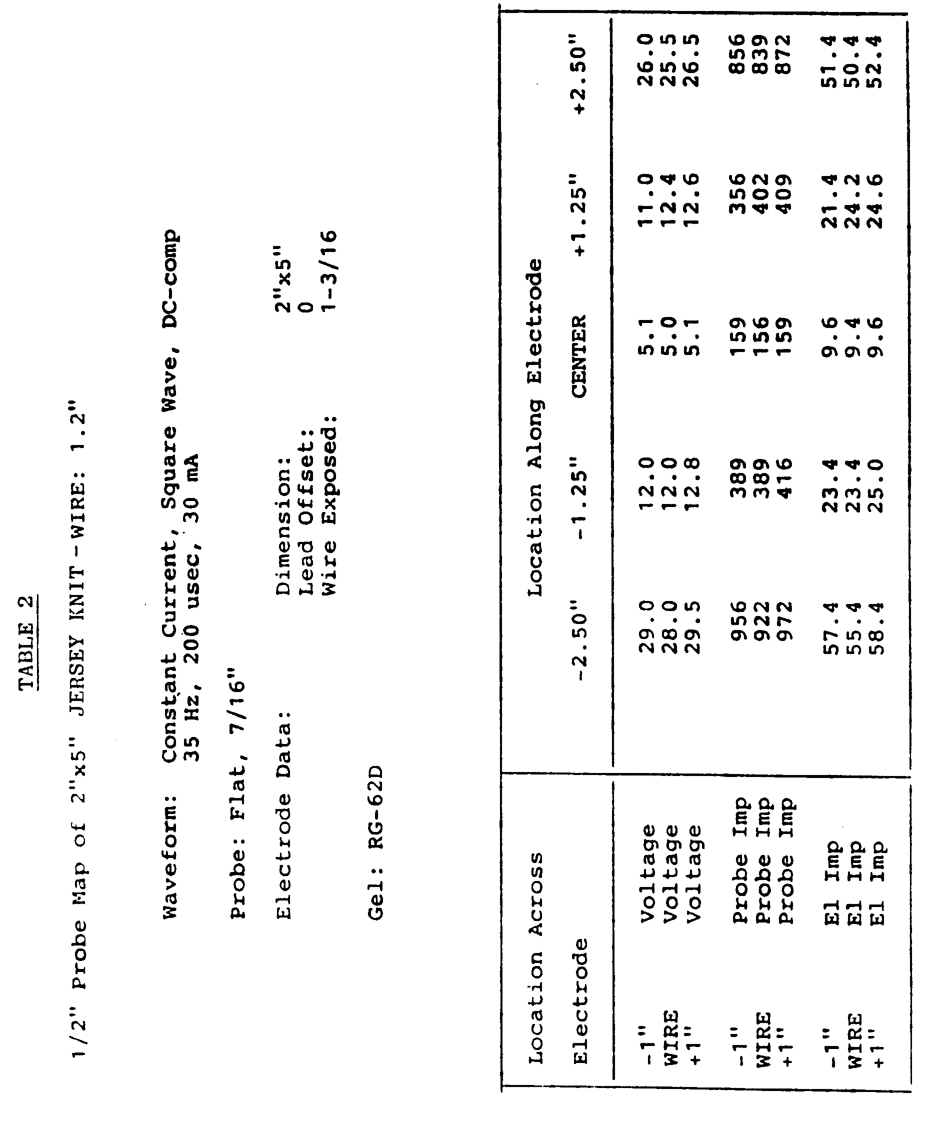

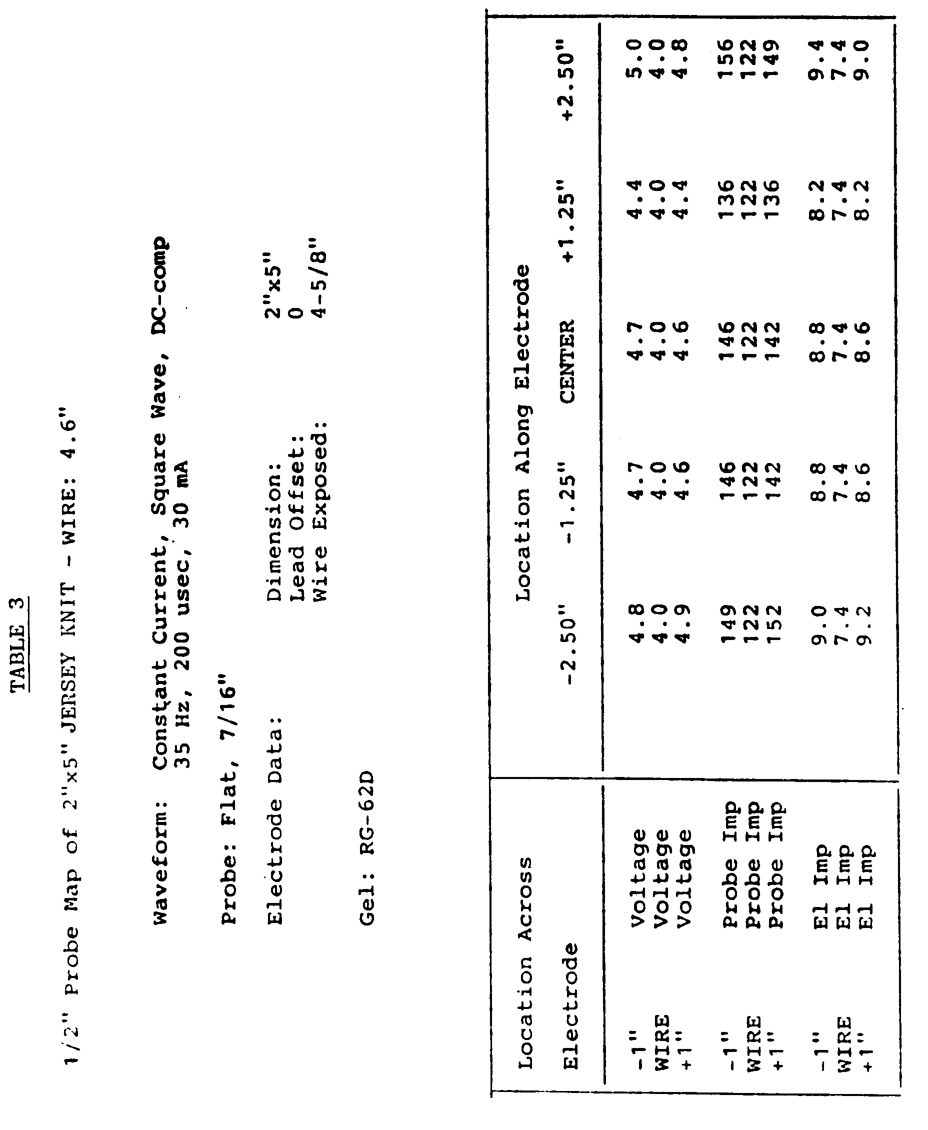

It can be easily seen from an examination of Figures 15 and 16 that the extended shunt causes significantly greater uniformity in impedance across the entire surface of the electrode.

Tables 4 and 5, corresponding Figures 17 and 18 show the impedance of an oval electrode, made in accordance with the present invention, utilizing a woven fabric with an exposed wire length of 1.2 inches and a jersey knit fabric with an exposed wire length of about 4 inches, respectively. As hereinabove pointed out, the woven fabric has an inherent uniform impedance gradient. However, as set forth in Table 1, a jersey knit fabric does not. In accordance with the present invention, uniform impedance can be achieved utilizing the knit fabric as shown by the data in Tables 4 and 5 and corresponding plots shown in Figures 17 and 18.

It has been further discovered that uniform impedance may be achieved by both varying the length of exposed shunt wire to control the longitudinal impedance gradient and further, controlling the transverse impedance gradient by varying the ratio of stainless steel to polyester in the yarn, and by changing the knitting patterns. For example, the diamond knit shown in Figure 4 has up to nine layers of yarn which result in approximately 5 to 1 X-Y impedance ratio, while a conventional jersey knit has only two layers giving it a 10 to 1 impedance ratio.

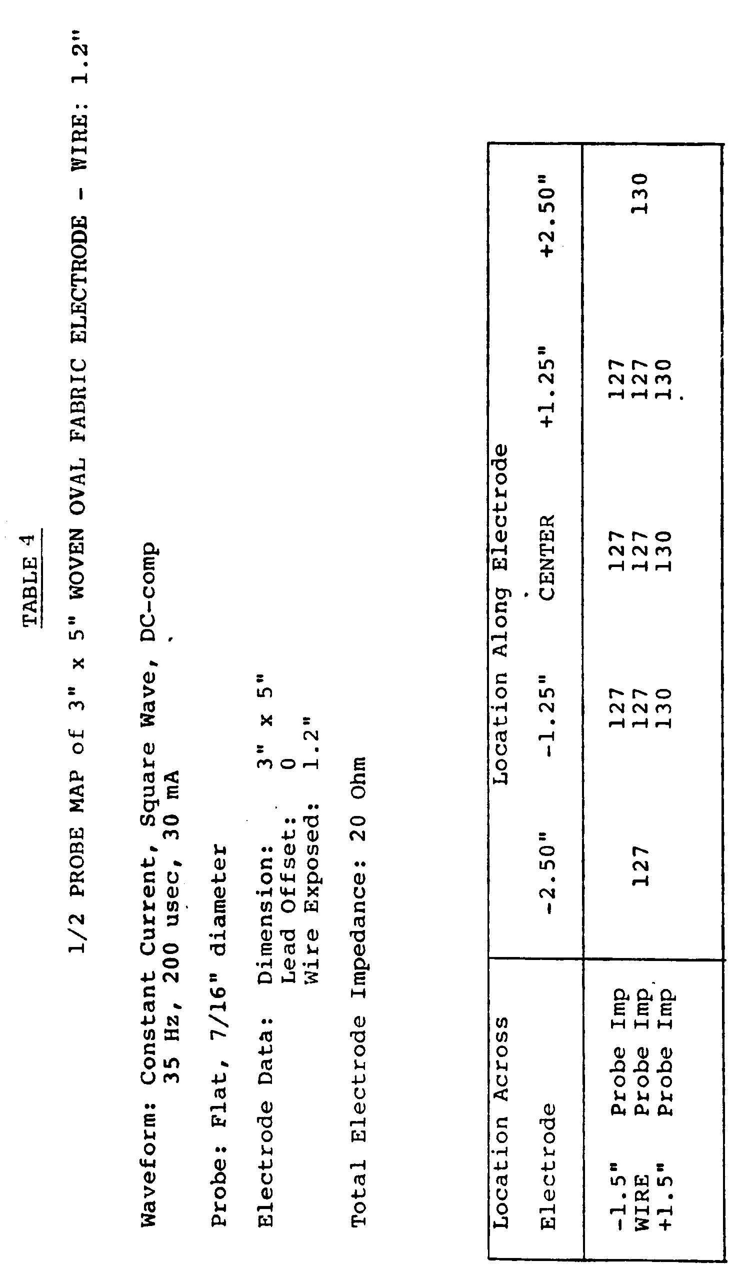

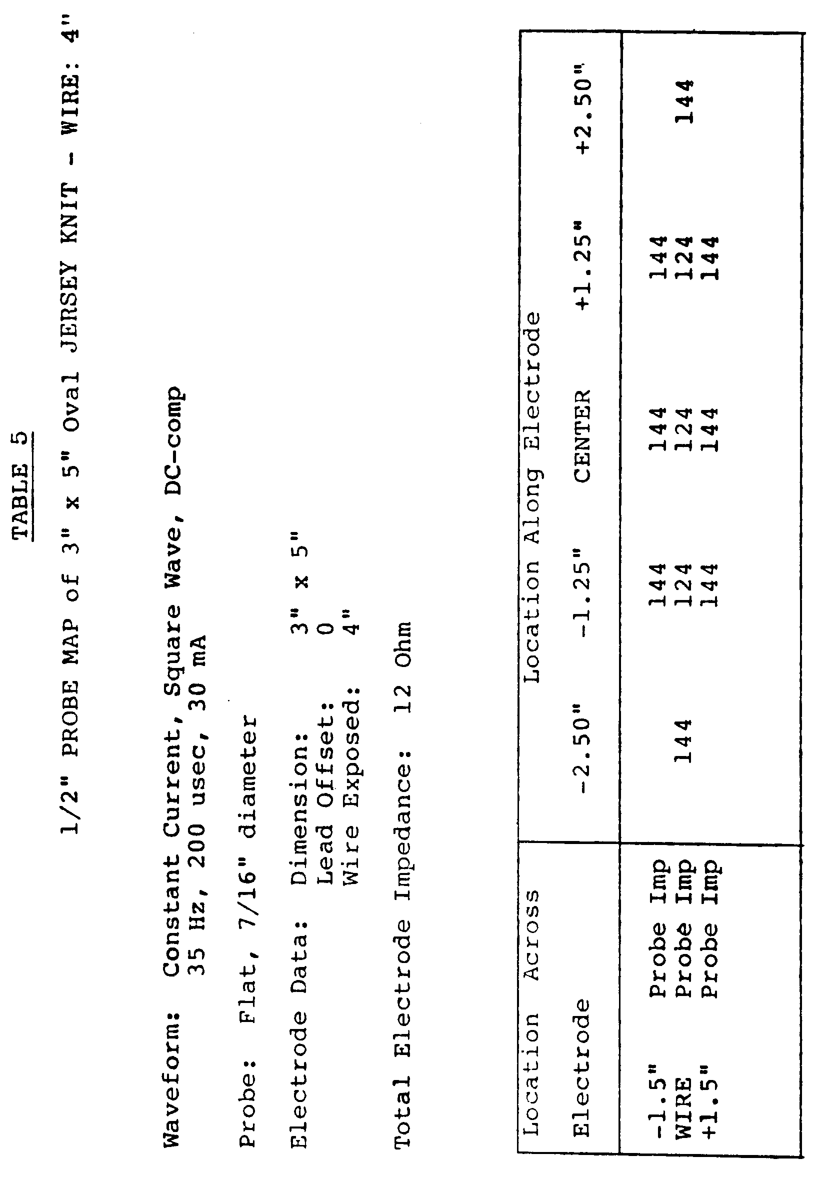

Although hereinabove described in terms of knit or woven fabrics, the conductive fabric may also be any conductive material, such as for example, a conductive plastic. The present invention is particularly suited for use with conductive material having different electrical resistivity in different directions therealong. In this case, the use of shunts as hereinbefore described enables the fabrication of an electrode with uniform resistivity. Importantly, as shown in Figure 18, the resistivity of an electrode can be tailored so that the current density across the electrode can taper, or decline, in a preselected manner to reduce or eliminate discomfort to the user due to large discontinuity in the edge current density as has been experienced by prior electrodes. (See Proceeding of IEEE Engineering in Medicine and Biology Society, Sept. 15-17, 1984, p. 187-190, Reddy, C.H. and Webster, J. G.).

Although there has been hereinabove described a specific arrangement of a flexible transcutaneous electrical nerve and/or muscle stimulation electrode in accordance with the invention for the purpose of illustrating the manner in which the invention may be used to advantage, it will be appreciated that the invention is not limited thereto. Accordingly, any and all modifications, variations, or equivalent arrangements which may occur to those skilled in the art, should be considered to be within the scope of the invention as defined in the appended claims.

## Claims

1. A flexible transcutaneous electrical nerve and/or muscle stimulation electrode (10, 44, 60, 70) having a conductive fabric (22) comprising conductive fibers (46) for both enabling the conductive fabric (22) to be stretched, and for directly coupling electrical signals to a patient's skin, said conductive fabric (22) comprising an array of conductive fibers (46) with interstitial areas (50) therebetween, and a flexible solid conductive adhesive (24) disposed within said interstitial areas (50) and on one side of said conductive fabric (22) for both adhering the flexible transcutaneous electrical nerve and/or muscle stimulation electrode (10, 44, 60, 70) to the skin of a patient and providing an electrical conducting contact therebetween, with an electrical lead wire (30) adapted for interconnection with an electrical stimulation device for providing electrical signals to said conductive fabric (22), and a non-conductive sheet (32) disposed on another side of said conductive fabric (22) for supporting the electrical shunt (66) along the conductive fabric (22) and for preventing undesired electrical contact with the conductive fabric (22), characterized by said conductive fabric (22) having different electrical resistivity in different directions therealong and by an electrical shunt (66) disposed along said conductive fabric (22) for causing more uniform resistance between any two equally spaced apart points on said conductive fabric (22) than without said electrical shunt (66).

2. The flexible stimulation electrode according to claim 1, further characterized in that the electrical shunt (66, 74) includes a plurality of strands (88) disposed in a vein-like manner having a dichotomous venation.

3. The flexible stimulation electrode according to Claim 1, further characterized in that the electrical shunt (66, 74) includes a plurality of strands (90) disposed in a vein-like manner having a pinnate reticulate venation.

4. The flexible stimulation electrode according to claim 1, further characterized in that the electrode shunt (66, 74) includes a plurality of strands (92) disposed in a vein-like manner having a palmate reticulate venation.

5. The flexible stimulation electrode according to claim 1, further characterized in that electrical shunt (66, 74) includes a plurality of strands (94) disposed in a vein-like manner having a parallel venation.

6. The flexible stimulation electrode according to Claims 2, 3, 4, or 5, further characterized in that the electrical shunt (66, 74) is disposed on an opposite side of said conductive fabric (22) and is held against said opposite side in an electrically conductive manner by the flexible solid conductive adhesive (24) disposed in said interstitial area (50).

7. The flexible stimulation electrode according to claim 6, further characterized in that the electrode shunt (66, 74) comprises a plurality of strands (88, 90, 92,94) of stainless steel wire having a diameter of about 8 microns.

8. The flexible stimulation electrode according to claim 1, further characterized in that the electrode shunt (66, 74) equalizes electrical resistance between any two opposing points along the perimeter of the conductive fabric (22).

9. The flexible stimulation electrode according to claim 8, further characterized by the fact that the said conductive fabric (22) comprises a knit of conductive fibers (46) having a higher resistivity along a longitudinal direction of knit than along a transverse direction of knit.

10. The flexible stimulation electrode according to claim 1, further characterized by the fact that the resistivity along the longitudinal direction of knit is up to about 20 times the resistivity along the transverse direction of knit.

11. The flexible stimulation electrode according to claim 1, further characterized by the fact that the conductive fabric (22) overall shape comprises a shape resembling a bow-tie (60).

12. The flexible stimulation electrode according to claim 1, further characterized by the fact that conductive fabric (22) overall shape comprises an oval shape (70).

13. A flexible transcutaneous electrical nerve and/or muscle stimulation electrode (10, 44, 60, 70) having a conductive material for coupling electrical signals to a patient's skin, and a flexible solid conductive adhesive (24) disposed on one side of said conductive material (22) for both adhering the flexible transcutaneous electrical nerve and/or muscle stimulation electrode (10, 44, 60, 70) to the skin of a patient and providing an electrical conducting contact therebetween, with an electrical lead wire (30) adapted for interconnection with an electrical stimulation device for providing electrical signals to said conductive material (22), and a non-conductive sheet (33) disposed on another side of said conductive material (22) for preventing undesired electrical contact with the conductive material (22), characterized by said conductive material (22) having different electrical resistivity in different directions therealong and by an electrical shunt (66, 74) disposed along said conductive material for causing more uniform resistance between any two equally spaced apart points on said conductive material than without said electrical shunt (66, 74).

14. The flexible stimulation electrode according to claim 13, further characterized in that the conductive material (22) has a different electrical resistivity in different directions therealong.

15. The flexible stimulation electrode according to claim 13, further characterized in that the electrical shunt (66, 74) includes a plurality of strands (88) disposed in a vein-like manner having a dichotomous venation.

16. The flexible stimulation electrode according to claim 13, further characterized in that the electrical shunt (66, 74) includes a plurality of strands (90) disposed in a vein-like manner having a pinnate reticulate venation.

17. The flexible stimulation electrode according to claim 13, further characterized in that the electrode shunt (66, 74) includes a plurality of strands (92) disposed in a vein-like manner having a palmate reticulate venation.

18. The flexible stimulation electrode according to claim 13, further characterized in that the electrical shunt (66, 74) includes a plurality of strands (94) disposed in a vein-like manner having a parallel venation.

19. The flexible stimulation electrode according to claim 1 or 13, further characterized in that the electrical shunt (66, 74) is terminated a selected distance from an edge of the conductive fabric (22) in order to substantially reduce unwanted high current density at the conductive fabric edge.

## Patentansprüche

1. Flexible transkutane elektrische Nerven- und/oder Muskelstimulationselektrode (10, 44, 60, 70) mit einem leitfähigen Tuch (22) mit leitfähigen Fasern (46), um zu ermöglichen, daß sowohl die leitfähige Textur (22) gestreckt als auch elektrische Signale direkt zu der Haut eines Patienten gekoppelt werden können, wobei das leitfähige Tuch (22) eine Anordnung von leitfähigen Fasern (46) mit Zwischenraumbereichen (50) dazwischen aufweist und ein flexibles festes leitfähiges Klebemittel (24) in den Zwischenraumbereichen (50) und auf einer Seite des leitfähigen Tuches (22) angebracht ist, um sowohl die flexible transkutane elektrische Nerven- und/oder Muskelstimulationselektrode (10, 44, 60, 70) an der Haut eines Patienten zu befestigen als auch einen elektrischen Leitkontakt zwischen diesen bereitzustellen, mit einem elektrischen Leitungsdraht (30), welcher zum Verbinden mit einer elektrischen Stimulationsvorrichtung geeignet ist, um elektrische Signale an das leitfähige Tuch (22) zu liefern, und einer nicht leitfähigen Folie (32), die auf einer anderen Seite des leitfähigen Tuches (22) angeordnet ist, um den elektrischen Nebenschluß (66) entlang des leitfähigen Tuches (22) zu stützen und um einen unerwünschten elektrischen Kontakt mit dem leitfähigen Tuch (22) zu vermeiden, dadurch gekennzeichnet, daß das leitfähige Tuch (22) einen unterschiedlichen elektrischen Widerstand entlang unterschiedlichen Richtungen von ihm hat und daß ein elektrischer Nebenschluß (66) entlang des leitfähigen Tuches (22) angeordnet ist, um einen Widerstand zwischen zwei beliebigen, gleich voneinander beabstandeten Punkten an dem leitfähigen Tuch (22) zu bewirken, der gleichförmiger ist als ohne den elektrischen Nebenschluß (66).

2. Flexible Stimulationselektrode nach Anspruch 1, weiterhin dadurch gekennzeichnet, daß der elektrische Nebenschluß (66, 74) eine Mehrzahl von Adern (88) aufweist, die in geäderter Weise angeordnet sind und ein dichotomes Geäder haben.

3. Flexible Stimulationselektrode nach Anspruch 1, weiterhin dadurch gekennzeichnet, daß der elektrische Nebenschluß (66, 74) eine Mehrzahl von Adern (90) aufweist, die in geäderter Weise angeordnet sind und ein gefiedertes netzförmiges Geäder haben.

4. Flexible Stimulationselektrode nach Anspruch 1, weiterhin dadurch gekennzeichnet, daß der Elektroden-Nebenschluß (66, 74) eine Mehrzahl von Adern (92) aufweist, die in geäderter Weise angeordnet sind und ein handförmiges netzförmiges Geäder haben.

5. Flexible Stimulationselektrode nach Anspruch 1, weiterhin dadurch gekennzeichnet, daß der elektrische Nebenschluß (66, 74) eine Mehrzahl von Adern (94) aufweist, die in geäderter Weise angeordnet sind und ein paralleles Geäder haben.

6. Flexible Stimulationselektrode nach Anspruch 2, 3, 4 oder 5, weiterhin dadurch gekennzeichnet, daß der elektrische Nebenschluß (66, 74) an einer gegenüberliegenden Seite des leitfähigen Tuches (22) angeordnet ist und gegen die gegenüberliegende Seite in einer elektrisch leitenden Weise durch das flexible feste leitfähige Klebemittel (24) gehalten wird, das in dem Zwischenraumbereich (50) angeordnet ist.

7. Flexible Stimulationselektrode nach Anspruch 6, weiterhin dadurch gekennzeichnet, daß der Elektroden-Nebenschluß (66, 74) eine Mehrzahl von Adern (88, 90, 92, 94) aus Draht aus korrosionsbeständigem Stahl mit einem Durchmesser von ca. 8 Mikrometer aufweist.

8. Flexible Stimulationselektrode nach Anspruch 1, weiterhin dadurch gekennzeichnet, daß der Elektroden-Nebenschluß (66, 74) einen elektrischen Widerstand zwischen zwei beliebigen gegenüberliegenden Punkten entlang des Umfanges des leitfähigen Tuches (22) ausgleicht.

9. Flexible Stimulationselektrode nach Anspruch 8, weiterhin gekennzeichnet durch die Tatsache, daß das leitfähige Tuch (22) ein Gewirk aus leitfähigen Fasern (46) aufweist, deren Widerstand entlang einer Längsrichtung des Gewirks höher ist als entlang einer Querrichtung des Gewirks.

10. Flexible Stimulationselektrode nach Anspruch 1, weiterhin gekennzeichnet durch die Tatsache, daß der Widerstand in Längsrichtung des Gewirks bis zu etwa 20 mal größer ist als der Widerstand in Querrichtung des Gewirks.

11. Flexible Stimulationselektrode nach Anspruch 1, weiterhin gekennzeichnet durch die Tatsache, daß die Gesamtform des leitfähigen Tuches (22) eine Form aufweist, die die einer Fliege (60) (=besondere Krawatte) ähnelt.

12. Flexible Stimulationselektrode nach Anspruch 1, weiterhin gekennzeichnet durch die Tatsache, daß die Gesamtform des leitfähigen Tuches (22) eineovale Form (70) hat.

13. Flexible transkutane elektrische Nerven- und/oder Muskelstimulationselektrode (10, 44, 60, 70) mit einem leitfähigen Material zum Koppeln von elektrischen Signalen mit der Haut eines Patienten und einem flexiblen festen leitfähigen Klebemittel (24), das auf einer Seite des leitfähigen Materials (22) angeordnet ist, um sowohl die flexible transkutane elektrische Nerven- und/oder Muskelstimulationselektrode (10, 44, 60, 70) an der Haut eines Patienten zu befestigen und einen elektrischen Leitkontakt zwischen diesen bereitzustellen, mit einem elektrischen Leitungsdraht (30), welcher zum Verbinden mit einer elektrischen Stimulationsvorrichtung geeignet ist, um elektrische Signale an das leitfähige Material (22) zu liefern, und einer nicht leitfähigen Folie (33), die auf einer anderen Seite des leitfähigen Materials (22) angeordnet ist, um einen unerwünschten elektrischen Kontakt mit dem leitfähigen Material (22) zu verhindern, dadurch gekennzeichnet, daß das leitfähige Material (22) einen unterschiedlichen elektrischen Widerstand entlang unterschiedlichen Richtungen von ihm hat und daß ein elektrischer Nebenschluß (66, 74) entlang des leitfähigen Materials angeordnet ist, um einen Widerstand zwischen zwei beliebigen gleich voneinander beabstandeten Punkten an dem leitfähigen Material zu bewirken, der gleichförmiger ist als ohne den elektrischen Nebenschluß (66, 74).

14. Flexible Stimulationselektrode nach Anspruch 13, weiterhin dadurch gekennzeichnet, daß das leitfähige Material (22) einen unterschiedlichen elektrischen Widerstand in verschiedenen Richtungen von ihm hat.

15. Flexible Stimulationselektrode nach Anspruch 13, weiterhin dadurch gekennzeichnet, daß der elektrische Nebenschluß (66, 74) eine Mehrzahl von Adern (88) aufweist, die in geäderter Weise angeordnet sind und ein dichotomes Geäder haben.

16. Flexible Stimulationselektrode nach Anspruch 13, weiterhin dadurch gekennzeichnet, daß der elektrische Nebenschluß (66, 74) eine Mehrzahl von Adern (90) aufweist, die in geäderter Weise angeordnet sind und ein gefiedertes netzförmiges Geäder haben.

17. Flexible Stimulationselektrode nach Anspruch 13, weiterhin dadurch gekennzeichnet, daß der Elektroden-Nebenschluß (66, 74) eine Mehrzahl von Adern (92) aufweist, die in geäderter Weise angeordnet sind und ein handförmiges netzförmiges Geäder haben.

18. Flexible Stimulationselektrode nach Anspruch 13, weiterhin dadurch gekennzeichnet, daß der elektrische Nebenschluß (66, 74) eine Mehrzahl von Adern (94) aufweist, die in geäderter Weise angeordnet sind und ein paralleles Geäder haben.

19. Flexible Stimulationselektrode nach Anspruch 1 oder 13, weiterhin dadurch gekennzeichnet, daß der elektrische Nebenschluß (66, 74) in einem gewählten Abstand von einem Rand des leitfähigen Tuches (22) endet, um eine unerwünschte hohe Stromdichte an dem Rand des leitfähigen Tuches wesentlich zu reduzieren.

## Revendications

1. Électrode flexible de stimulation électrique transcutanée d'un nerf et/ou d'un muscle (10, 44, 60, 70) comportant un tissu conducteur (22) comprenant des fibres conductrices (46) pour, à la fois, permettre au tissu conducteur (22) d'être étiré et coupler directement des signaux électriques à la peau d'un patient, ledit tissu conducteur (22) comprenant un groupement de fibres conductrices (46) avec, entre elles, des zones interstitielles (50), et un adhésif conducteur plein flexible (24) disposé à l'intérieur desdites zones interstitielles (50) et sur une première face dudit tissu conducteur (22) pour, à la fois, faire adhérer l'électrode flexible de stimulation électrique transcutanée d'un nerf et/ou d'un muscle (10, 44, 60, 70) à la peau d'un patient et assurer un contact conducteur électrique entre elles, avec un fil conducteur électrique (30) conçu pour interconnexion avec un dispositif de stimulation électrique pour fournir des signaux électriques audit tissu conducteur (22), ainsi qu'une feuille non conductrice (32) disposée sur l'autre face dudit tissu conducteur (22) pour supporter le shunt électrique (66) le long du tissu conducteur (22) et pour empêcher un contact électrique indésirable avec le tissu conducteur (22), caractérisée par le fait que, le long dudit tissu conducteur (22), il y a une résistivité électrique différente dans des directions différentes et par un shunt électrique (66) disposé le long dudit tissu conducteur (22) pour donner, entre deux points quelconques également espacés l'un de l'autre sur ledit tissu conducteur (22), une résistance plus uniforme qu'en l'absence dudit shunt électrique (36).

2. Électrode flexible de stimulation selon la revendication 1, caractérisée en outre par le fait que le shunt électrique (66, 74) contient une pluralité de brins (68) disposés en mode ramifié avec ramification dichotomique.

3. Électrode flexible de stimulation selon la revendication 1, caractérisée en outre par le fait que le shunt électrique (66, 74) contient une pluralité de brins (90) disposés en mode ramifié avec une ramification réticulée pennée.

4. Électrode flexible de stimulation selon la revendication 1, caractérisée en outre par le fait que le shunt électrique (66, 74) contient une pluralité de brins (92) disposés en mode ramifié avec une ramification réticulée palmée.

5. Électrode flexible de stimulation selon la revendication 1, caractérisée en outre par le fait que le shunt électrique (66, 74) contient une pluralité de brins (94) disposés en mode ramifié avec une ramification en parallèle.

6. Électrode flexible de stimulation selon les revendications 2, 3,4 ou 5, caractérisée en outre par le fait que le shunt électrique (66, 74) est disposé sur une face opposée dudit tissu conducteur (22) et qu'il est maintenu contre la dite face opposée, de façon électriquement conductrice, par l'adhésif conducteur plein flexible (24) disposé dans ladite zone interstitielle (50).

7. Électrode flexible de stimulation selon la revendication 6, caractérisée en outre par le fait que ledit shunt d'électrode (66, 74) contient une pluralité de brins (88, 90, 92, 94) de fil d'acier inoxydable d'un diamètre d'environ 8 microns.

8. Électrode flexible de stimulation selon la revendication 1, caractérisée en outre, par le fait que le shunt d'électrode (66, 74) rend égales les résistances électriques entre deux points opposés quelconques le long du périmètre du tissu conducteur (22).

9. Électrode flexible de stimulation selon la revendication 8, caractérisée en outre par le fait que ledit tissu conducteur (22) comprend un tricot de fibres conductrices (46) présentant une résistivité plus élevée selon une direction longitudinale du tricot que selon une direction transversale du tricot.

10. Électrode flexible de stimulation selon la revendication 1, caractérisée en outre par le fait que la résistivité selon la direction longitudinale du tricot vaut jusqu'à environ 20 fois la résistivité selon la direction transversale du tricot.

11. Électrode flexible de stimulation selon la revendication 1, caractérisée en outre par le fait que la forme d'ensemble du tissu conducteur (22) comporte une forme ressemblant à un noeud papillon (60).

12. Électrode flexible de stimulation selon la revendication 1, caractérisée en outre par le fait que la forme d'ensemble du tissu conducteur (22) comporte une forme ovale (70).

13. Électrode flexible de stimulation électrique transcutanée d'un nerf et/ou d'un muscle (10, 44 60, 70) comportant un matériau conducteur pour coupler des signaux électriques à la peau d'un patient, et un adhésif conducteur plein flexible (24) disposé sur une première face dudit matériau conducteur (22) pour, à la fois, faire adhérer l'électrode flexible de stimulation électrique transcutanée d'un nerf et/ou d'un muscle(10, 44, 60, 70) à la peau d'un patient et assurer un contact conducteur électrique entre eux, avec un fil conducteur électrique (30) conçu pour interconnexion avec un dispositif de stimulation électrique pour fournir les signaux électriques audit matériau conducteur (22), ainsi qu'une feuille non-conductrice (33) disposée sur l'autre face dudit matériau conducteur (22) pour empêcher un contact électrique indésirable avec le matériau conducteur (22), caractérisée par le fait que, le long dudit matériau conducteur (22), il y a une résistance électrique différente dans des directions différentes et par un shunt électrique (66, 74) disposé le long dudit matériau conducteur pour donner, entre deux points quelconques également espacés l'un de l'autre sur ledit matériau conducteur, une résistance plus uniforme qu'en l'absence dudit shunt électrique (66, 74).

14. Électrode flexible de stimulation selon la revendication 13, caractérisée en outre par le fait que, le long du matériau conducteur (22), il y a une résistivité électrique différente dans des directions différentes.

15. Électrode flexible de stimulation selon la revendication 13, caractérisée en outre par le fait que le shunt électrique (66, 74) contient une pluralité de brins (68) disposés en mode ramifié avec ramification dichotomique.

16. Électrode flexible de stimulation selon la revendication 13, caractérisée en outre par le fait que le shunt électrique (66, 74) contient une pluralité de brins (90) disposés en mode ramifié avec une ramification réticulée pennée.

17. Électrode flexible de stimulation selon la revendication 13, caractérisée en outre par le fait que le shunt électrique (66, 74) contient une pluralité de brins (92) disposés en mode ramifié avec une ramification réticulée palmée.

18. Électrode flexible de stimulation selon la revendication 13, caractérisée en outre par le fait que le shunt électrique (66, 74) contient une pluralité de brins (94) disposés en mode ramifié avec une ramification en parallèle.

19. Électrode flexible de stimulation selon la revendication 1 ou 13, caractérisée en outre par le fait que le shunt électrique (66, 74) se termine à une distance choisie d'un bord du tissu conducteur (22) pour réduire substantiellement une densité de courant de haute valeur non désirée au bord du tissu conducteur.
